Europäisches Patentamt

European Patent Office

Office européen des brevets

(11) Publication number: 0 368 674
A2

## (12) EUROPEAN PATENT APPLICATION

(21) Application number: 89311660.8

(51) Int. Cl.5: G01N 33/543

(22) Date of filing: 10.11.89

(30) Priority: 11.11.88 JP 286071/88
16.11.88 JP 290977/88

(43) Date of publication of application:
16.05.90 Bulletin 90/20

(84) Designated Contracting States:
DE ES FR IT

(71) Applicant: SANYO CHEMICAL INDUSTRIES, LTD.
No.11-1, Ichinohashi-nomoto-cho
Higashiyama-ku
Kyoto-shi Kyoto-fu(JP)

(72) Inventor: Sakata, Kazuto
4-18, Imakumano-Minamidani-cho
Higashiyama-ku
Kyoto(JP)
Inventor: Kikutake, Jun-ichiro
6-16-19, Yamatedai
Ibaraki-shi Osaka-fu(JP)
Inventor: Sigiura, Masakazu
151-4, Mukaijima-ninomaru-cho Fushimi-ku
Kyoto(JP)

(74) Representative: Marlow, Nicholas Simon et al
Reddie & Grose 16, Theobalds Road
London WC1X 8PL(GB)

### (54) Immunoassay and test kits therefor.

(57) Immunoassays of improved sensitivity, even when monoclonal antibodies are of poor affinity, are disclosed.

An immunoassay method of the invention comprises the steps of:
(1) reacting a material to be measured, an immobilized monoclonal antibody (1st antibody) recognizing said material, a monoclonal antibody (2nd antibody) recognizing said material and falling within a different class or subclass fro the 1st antibody, and a labelled antibody (3rd antibody) recognizing the class or subclass of the 2nd antibody to obtain an immuno-complex, and (2) detecting or determining the labelling fragment.

In another aspect, the invention provides immunoassay method comprising the steps of: (1) reacts a material to be measured, a protein (the 1st binding protein) to bind specifically to said material, an immobilized protein (the 2nd binding protein) comprising the rest of the 1st binding protein, a part of which having been excised away, and a labelled antibody razing all or part of the excised fragment of the 1st bonding protein to obtain an immuno-complex, and (2) detecting or determining the labelling fragment.

# IMMUNOASSAY METHODS AND TEST KITS THEREFOR

This invention relates to immunoassay methods and kits therefor. More particularly, it relates to immunoassay methods and test kits for detecting or determining materials to be measured in testing samples (for example, those in serum or other body fluids associated with a wide variety of physiological disorders) by use proteins (such as monoclonal antibodies) binding specifically to said materials and antibodies recognizing these proteins.

As immunoassay methods, there have been known those comprising reacting an immobilized monoclonal antibody, antigenic substance a a labelled monoclonal antibody, followed by measuring the amount of the label (such as JPN Patent Lay-open Nos. 86051/1982, 118159/1982 and 79455/1982).

These known immunoassay using monoclonal antibodies, however, have drawbacks, such that sufficient sensitivity is not obtained when the monoclonal antibodies have poor affinity.

According to the invention there is provided an immunoassay method comprising the steps of:
(1) reacting a material to be measured, an immobilized monoclonal antibody (the 1st antibody) recognizing said material, a monoclonal antibody (the 2nd antibody) recognizing said material and falling within a different class or subclass from the 1st antibody, and a labelled antibody (the 3rd antibody) recognizing the class or subclass of the 2nd antibody to obtain an immuno-complex, and (2) detecting or determining the labelling fragment; or by an immunoassay method arising the steps of:
(1) reacting a material to be measured, a protein (the 1st binding protein) to bind specifically to said material, an immobilized protein (the 2nd binding protein) comprising the rest of the 1st protein, a part of which having been used away, and a labelled antibody recognizing all or a part of the excised fragment of the 1st bonding protein to obtain an immuno-complex; and (2) detecting or determining the labelling fragment.

In the drawings,
Fig. 1 shows CA19-9 standard curves obtained in Example 1 and Comparative Example 1;
Fig. 2 shows CEA standard curves obtained in Example 2 and Comparative Example 2;
Fig. 3 shows HBs antigen standard curves obtained in Example 3 and Comparative Example 3;
Fig. 4 shows HCG standard curves obtained in Example 4 and Comparative Example 4;
Fig. 5 shows CA19-9 standard curves obtained in Example 5 and Comparative Example 5; and
Fig. 6 shows HBs antigen standard curves obtained in Example 6 and Comparative Example 6.

Suitable materials to be measured include, for example, antigenic substances, enzymes, and the like. Illustrative examples of suitable materials to be measured according to the present invention, are

1) hormones, such as insulin, human chorionic gonadotropin beta-subunit (HCG-beta) growth hormone, thyroid stimulating hormone (TSH), thyroxine, triiodothyronine, gastrin, glucagon, somatostatin, LH, FSH, prolactine, and the like;

2) enzymes, such as elastase, amylase, proteiase, lipase, ribonuclease, and the like;

3) serum proteins, such as IgG, IgA, IgM, IgE, IgD, glycoproteins, $beta_2$-microglobulin, TBG, glycolipids, IAP, $C_3$, $C_4$, $C_5$, CRP, $alpha_2$-microglobulin, HPL, transferrin, albumin, and the like;

3)tumor-associated antigens, such as carcinoembryonic antigen (CEA), alpha-fetoprotein (AFP), ferritin, POA, CA-19-9, CA125, and the like;

4)DNA binding protein factors;

5)cytokines, such as interferon, interleukin 1, inter-leukin 2 and the like; and

6)pathogens (pathogenic bacteria, viruses, parasites or protozoas, causing various disease), such as eumycetes, streptococcus, hepatitis viruses (such as hepatitis B virus), rubella virus, herpes virus, AIDS virus, toxoplasma Niccole-Manceaux, plasmodium, entameoeba histolyticaschaudinn, and the like.

Among these, preferred are hormones and tumor associated antigens, for which high sensitivity measuring systems are desired.

In an aspect of this invention, an immobilized monoclonal antibody recognizing said material to be measured is used as the 1st antibody.

Suitable 1st antibodies include, for example, cell-originated antibodies obtained by hybrid cells according to the method disclosed in literatures [such as G.Kohler, C.Milstein: Natur, Vol.256,p.495-(1975)]. Basically, it involves immunizing a mouse or other suitable animal (such as rabbit, goat, sheep, guinea pig and the like) with an immunogen, fusing antibody-producing cells taken from the animal with myeloma cells (originated from a mouse or other suitable animal), and culturing or injecting intraperitoneally the resulting hybrid cells into the animal to obtain ascites. These antibodies may be purified by known methods, such as ammonium-sulfate precipitation, DEAE-cellulose chromatography, affinity chromatography, gel filtration chromatography and the like. Such monoclonal antibodies, produced from antibody-producing cells of the same clone, are perfectly same, and are believed to have quite the same specificity towards the materials to be measured and belong to quite the same class and subclass.

Among 1st antibodies, preferred are monoclonal antibodies, obtained using, as the immunogen, hormones (particularly insulin, HCG-beta and TSH) and tumor-associated antigens (especially CEA, AFP and CA-19-9).

Said 1st antibody, in the invention, can be immobilized on a substrate, such as inorganic or organic carrier (insoluble solid).

Suitable carriers include inorganic carriers, for example,siliceous materials [such as glass (porous glass, frosted glass and so on ), silica (silica gel, colloidal silica), bentonite, wollastonite, cordierite and the like], and nonsiliceous metal oxides [such as alumina, spinel, apatite, hydroxy apatite, titania, zirconia and magnetic substances (such as iron oxides, ferrite, nickel oxides, cobalt oxides and the like )]; and organic carriers, for instance, plastics [such as polystyrene, and derivatives thereof, such as poly(amino-styrene); acrylic polymers, such as polyacrylonitrile; polymethacrylates, such as polymethylmethacrylate; polyolefines, such as polyethylene, polypropyrene, polybutene and polybutadiene; halogen-containing polymers, such as poly(vinyl chloride) and poly(vinylidene chloride); polyesters, such as poly(ethylene terephthalate); polyamides, such as nylon 6 and nylon 6,6; and so on]; and natural polymers, such as polysaccharides, cellulose, dextran, agarose, paper (such as filter paper), polypeptide, collagen and the like. Among these, preferred are glass, plastics, magnetic substances, cellulose and papers.

These carriers may be particulate in nature, varying from a finely divided powder to a coarse granular material (e.g. about 20 - about 100 mesh or more, U.S. Standard Sieve), or may be a shaped article, such as sheet or pellet or three-dimensional articles, such as beads, test tubes, trays, discs and so on. Among these, preferred are glass (particularly glass beads and glass test tubes) and plastics (plastic tubes and plastic trays). These carriers may be porous, or surface-modified by known methods, such as etching or frosting, chemical treatment, chemical coating and the like.

The 1st antibody can be immobilized by any known means, which can vary from simple adsorption to chemical coupling. Chemical coupling typically involves treating the carrier with one or more chemical compounds (silanes, polyisocyanates and the like), followed by contacting the treated carrier with an aqueous solution of 1st antibody. Adsorption usually involves contacting an aqueous solution of 1st antibody with the carrier for a time sufficient to permit the desired or maximum degree of immobilization. Examples of methods suitable for immobilization of lst antibody are those by physical adsorption or chemical coupling to glass with a silane coupling agent with or without a crosslinking agent, as described in U.S.Patents 4,280,992 and 3,652,761; and those by physical adsorption to plastics, as written in E.Engvall, J.Johnson, P.Parlman: Biochim. Biophys, Acta,251(1971)427-434. Among these methods, preferred are silane coupling and physical adsorption..

Illustrative examples of immobilized components 1st antibodies are anti-CA-19-9 antibodies, anti-HBs antibodies, anti-CEA antibodies and anti-HCG antibodies, supported on glass bead or plastic tray.

As the 2nd antibody, is used a monoclonal antibody recognizing said material to be measured and falling within a different class or subclass from the 1st antibody. Monoclonal antibodies as the 2nd antibodies can be obtained in the same manner as described above with respect to the 1st antibodies. Illustrative examples of suitable 2nd antibodies are monoclonal antibodies of IgM class when the 1st antibodies are monoclonal antibodies of IgG class, monoclonal antibodies of IgG class when the 1st antibodies are monoclonal antibodies of IgA class, monoclonal antibodies of IgG2a subclass when the 1st antibodies are monoclonal antibodies of IgG1 class. Among 2nd antibodies, preferred are monoclonal antibodies originated from the same animal species as the 1st antibodies.

As the labelled antibody (3rd antibody), there may be used an antibody specifically recognizing only the class or subclass of the 2nd antibody. Illustrative examples of suitable 3rd antibodies include anti-mouse IgG antibodies when the 1st antibodies are monoclonal antibodies of mouse IgA class and the 2nd antibodies are monoclonal antibodies of mouse IgG class, and anti-mouse IgG1 antibodies when the 1st antibodies are monoclonal antibodies of mouse IgG2a subclass and the 2nd antibodies are monoclonal antibodies of mouse IgG1 subclass.

Markers used for labelling the 3rd antibody include usually employed for this purpose. Illustrative examples of suitable markers include isotopes (radioisotopes, such as $H^3$, $I^{125}$ and the like), enzymes (such as peroxidase, beta-galactosidase, alkaline phosphatase, and the like), fluorescent substances (such as fluorescein isothiocyanate, rhodamine, europeum derivatives and the like), illuminant substances (such as isoluminole derivatives, N-methyl acridium derivatives and the like), and so on. Among these, preferred are isotopes (particularly $I^{125}$ ) and enzymes (particularly peroxidase), in view of easiness of labelling antibodies and high sensitivity.

Labelling may be performed by any known methods, for example, enzyme labelling by periodoic acid oxidation methods, as described in Nakane et al , J.Histochem.-Cytochem.$\underline{22}$,1084-(1974); and those described in S.-Yoshitake, M.Imagawa, E.Ishikawa, et al, J.Biochem.$\underline{92}$,1413-1424(1982).

In another embodiment of the present invention, a protein to bind specifically to said material to be measured is used as the 1st binding protein.

Suitable 1st binding proteins include monoclonal antibodies, polyclonal antibodies, enzymes, enzyme inhibitors, hormones, cytokines, receptors and lectin, capable of bonding specifically to materials to be measured. Examples of such proteins include those mentioned above. Illustrative 1st binding proteins include, for example, monoclonal antibodies for measuing antigenic substances; enzymes for enzyme inhibitors; enzyme inhibitors for enzymes; hormones for hormone receptors; hormone receptors for hormones; cytokines for cytokine receptors; cytokine receptors for cytokines; lectin for suger complexes. Among these, preferred are monoclonal antibodies.

Suitable 2nd binding proteins include ones obtainable by partly excising the 1st binding protein or the 1st antibody, followed by removing the excised fragment from the protein. Excising can be performed by known methods [such as those described in "Zoku-seikagaku-jikken-koza 5", "Research methods for Immuno-biochemistry", pages 89-101], with use of proteinases, such as pepsin, papain, trypsin, plasmin and the like. The resulting excised fragments can be removed by known methods, for example, gel filtration chromatography, protein A affinity chromatography [such as those described in "Zoku-seikagaku-jikken-koza 5", "Research methods for Immuno-biochemistry", page 100], to obtain the 2nd binding proteins.

The 2nd binding proteins can be immobolized in the same manner as described above.

Antibodies recognizing all or part of the excised fragments of the 1st bonding protein are used for labelling to obtain labelled antibodies. Illustrative examples of suitable antibodies for labelling include anti-mouse IgG Fc antibody, in case where the 1st bonding protein is mouse IgG monoclonal antibody and the excised fragment is Fc fragment; and anti-mouse IgA Fc antibody, when the 1st bonding protein is mouse IgA monoclonal antibody and the excised fragment is Fc fragment.

Markers and methods for labelling are the same as mentioned above.

Immuno-complexes can be produced by various methods such as the following methods :

(1) reacting a material (A) to be measured with an immobilized 1st antibody $(B_1)$ or an immobilized 2nd binding protein $(B_2)$, followed by reacting this (A) with a 2nd antibody $(C_1)$ or a 1st binding protein $(C_2)$, and then reacting this $(C_1)$ or $(C_2)$ with a 3rd antibody $(D_1)$ or a labelled antibody $(D_2)$;

(2) reacting simultaneously $(C_1)$ or $(C_2)$, (A) and $(B_1)$ or $(B_2)$, followed by reacting this $(C_1)$ or $(C_2)$ with $(D_1)$ or $(D_2)$;

(3) reacting simultaneously $(C_1)$ or $(C_2)$, a material (A), $(B_1)$ or $(B_2)$, and (D1) or (D2); and

(4) reacting $(B_1)$ or $(B_2)$ with (A), followed by reacting simultaneously (A), $(C_1)$ or $(C_2)$ and $(D_1)$ or $(D_2)$.

Method (1) can be carried out, for instance, as follows: $(B_1)$ or $(B_2)$ and (A) are incubated in a buffer, followed by washing the resulting solid material. Then, the solid material is transferred to a buffer containing $(C_1)$ or $(C_2)$,and after incubation to bind them, followed by washing the resulting solid material again. Thereafter, the solid material is transferred to a buffer containing $(D_1)$ or $(D_2)$, followed by incubating to bind them to obtain an immuno-complex.

In carrying out Method (2), $(C_1)$ or $(C_2)$, (A) and $(B_1)$ or $(B_2)$ are incubated in a buffer, followed by washing the resulting solid material. Then, the solid material is transferred to a buffer containing $(D_1)$ or $(D_2)$, followed by incubating to bind them to obtain an immuno-complex.

In Method (3), $(C_1)$ or $(C_2)$, (A), $(B_1)$ or $(B_2)$ and $(D_1)$ or $(D_2)$ are incubated in a buffer to bind them to obtain an immuno-complex.

In carrying out Method (4), $(B_1)$ or $(B_2)$ and (A) are incubated in a buffer to bind them, followed by washing the resulting solid material. Then, the solid material is transferred to a buffer containing $(C_1)$ or $(C_2)$ and $(D_1)$ or $(D_2)$, followed by incubating to bind them to obtain an immuno-complex.

In these methods, each reaction or incubation can be carried out under usual conditions, for example, 5 - 50°C, preferably 34 - 40°C, for 5 minutes - 2 days, preferably 5 - 30 minutes.

In these procedures, B/F separation and washing may be carried out in the usual way, for example, by removing the liquid under suction using an aspirator. Separated solid materials may be washed, for instance with 1 - 5 ml of a washing liquid (such as distilled water, normal saline solution, a phosphate buffer or the like), and the procedure may be repeated several times ( for example, twice - five times) to obtain an immuno-complex separated from unreacted materials.

Immnoassay methods for measuring the labelling fragment of immuno-complexes include any known methods, such as RIA using Isotopes, EIA using enzymes, FIA using fluorescent substances and ones using illuminant substances, and set forth. For instance, in case of using peroxidase as the marker, an immuno-complex are incubated (for example, 5 - 50°C, for 5 minutes - 2 days) in a solution of chromophoric substrate (such as o-phenylene diamine and hydrogenperoxide solution, 4-aminoantipyrine and hydrogenperoxide, and the like), followed by measuring chromophoric activity using a spectrophotometer to determine peroxidase activity.

Immunoassay according to the present invention can be applied to detection or determination of materials, even when monoclonal antibody is of poor affinity.

As another aspect, this invention provides test kits for immunoassay applicable to materials to be measured. Such kits of the invention include :

[I] those comprising :

1) an immobolized monoclonal antibody (1st antibody) recognizing said material,

2) a monoclonal antibody (2nd antibody) recognizing said material and falling within a different class or subclass from the 1st antibody, and

3) a labelled antibody (3rd antibody) recognizing the class or subclass of the 2nd antibody; and [II] those comprising :

1) a protein (the 1st binding protein) to bind specifically to said material,

2) an immobilized protein (the 2nd binding protein) comprising the rest of the 1st protein, a part of which having been excised away, and

3) a labelled antibody recognizing all or part of the excised fragment of the 1st binding protein.

Examples of [II] include those comprising :

1) a monoclonal antibody (the 1st antibody) to recognize antigenic substance to be measured,

2) an immobilized monoclonal antibody (the 2nd antibody) comprising the rest of the 1st antibody, a part of which having been excised away, and

3) a labelled antibody (3rd antibody) recognizing all or part of the excised fragment of the 1st antibody.

These test kits may contain optionally various auxiliaries, to make it convenient to use these kits. For example, in case where the 2nd antibody, the 1st binding protein, the 3rd antibody or the labelled antibody is solid, there may be used solvent therefor. Other examples are washing solution for immobilized solid used during the process for producing immuno-complexes; substrates for measuring enzyme activity and reaction terminators for enzyme reactions, in case of using enzyme as the marker.

According to the present invention, high sensitivity immonoassay can be obtained, even when monoclonal antibody of poor affinity is used. This invention can attain improvement of sensitivity without lowering precision; whereas known immnoassay using monoclonal antibodies, showing good precision, are of insufficient sensitivity in case where the monoclonal antibody has poor affinity.

Test kits of this invention, when applied as diagnostic reagents, can remarkably shorten measuring time, owing to improved sensitivity.

Thus, immunoassay methods and test kits of the invention are particularly useful for detection or determination of such materials, as tumor-associated antigens, hormones, virus and the like, contained in trace amount in serum or other body fluids, wherein high sensitivity measurement is required; and can attain reduction of detection time of these materials.

Having generally described the invention, a more complete understanding can be obtained by reference to certain specific examples, which are included for purposes of illustration only and are not intended to be limiting unless otherwise specified.

Example 1 [Measurement of CA19-9 ]

a) Producing monoclonal antibodies to CA19-9

Monoclonal antibodies to CA19-9 were produced as described in Hilary Koprowski,Zenon Steplewski,Kenneth Mitchell,Meenhard Helyn; Somatic Cell Genetics Vol.5,No.6,1979,PP.957-972. A BALB/c mouse was immunized by intravenous injection of $1 \times 10^6$ SW1116 cells (DAINIPPON PHARMACEUTICAL CO.,LTD.). One month later, the mouse was injected similarly and sacrificed after three days. After sacrifice, all spleen cells washed in RPMI1640 medium were mixed with $2 \times 10^7$ mouse myeloma cells (P3-NS1/1-Ag 4.1) and fused in 1ml of RPMI1640 medium included 42.5% polyethylene glycol 1540 and 7.5% dimethyl sulfoxide for 1 minute at 37°C. After 1 minute, the cells suspension was diluted gradually with 5ml of RPMI1640 medium. After the cells were separated centrifugally and washed, HAT medium (RPMI1640 medium containing hypoxanthine, aminopterin, thymidine and 10% fetal bovine serum) was added to amount to 20ml, and then the cells in HAT medium were seeded in 96 wells microplate. Two weeks later, culture media from wells containing growing hybrid cells were tested for the presence of antibody activity. The hybrid cells that had antibody activity were cloned repeatedly at limiting dilution, then two clones of hybrid cells that produced IgG1 subclass monoclonal antibodies and IgA class monoclonal antibodies were obtained. These two clones of hybrid cells were injected intraperitoneally into mice and ascites fluid was obtained after hybrid cells grew as ascites tumors. The monoclonal antibodies in ascites fluid were purified with affi-gel-protein A MAPS kit (BIO-RAD).

b) Producing glass beads conjugated monoclonal antibodies to CA19-9 (IgG1 subclass)

Monoclonal antibodies to CA19-9 (IgG1 subclass) were coated onto the surface of glass beads as described in U.S.Pat.No.3652761.

c) Producing peroxidase-labeled antibodies to mouse IgA

Antibodies to mouse IgA (Organon Teknika Corp.-Cappel Products) were conbined with peroxidase as described in S.Yoshitake, M.Imagawa,E.Ishikawa,et.al.; J.Biochem.,Vol.92,1982,pp.1413-1424. The peroxidase-labeled antibodies to mouse IgA were diluted to 1/10 - 1/5000 concentration with buffer containing 1% bovine serum albumin, and then used.

d) Producing CA19-9 standard solution

Culture medium was obtained by culturing SW1116 cells in RPMI 1640 medium containing 10% fetal bovine serum. Concentration of CA19-9 containing the culture medium was measured with IMNOCLONE CA19-9 kit (FUJIREBIO INC.). 30, 60, 120 and 240 unit/ml standard solutions were produced by diluting the culture media with buffer containing 1% bovine serum albumin.

e) Producing CA19-9 kits

One hundred glass beads conjugated monoclonal antibodies to CA19-9 (IgG1 subclass), 12 ml of 0.02M phosphate buffer containing monoclonal antibodies to CA19-9 (IgA class) at concentration of 10μg/ml, 60 ml of 0.02M phosphate buffer containing peroxidase-labeled antibodies to mouse IgA, 40 ml of 0.02M phosphate buffer containing 1% bovine serum albumin, 1 ml of each standard solution (30, 60, 120 and 240 unit/ml), 1000 ml of normal saline solution, 60 ml of substrate solution (o-phenylenediamine solution containing hydrogen peroxide) and 350 ml of 1.5N sulfuric acid solution werebottled and stoppered respectively. A CA19-9 kit consisted of these reagents.

f) Measuring CA19-9 standard solution

A glass bead conjugated monoclonal antibodies to CA19-9 (IgG1 subclass) was incubated with 50μl of 240 unit/ml standard solution, 100μl of

0.02M phosphate buffer containing monoclonal antibodies to CA19-9 (IgA class) at concentration of 10μg/ml and 300μl of 0.02M phosphate buffer containing 1% bovine serum albumin in a test tube for 15 minutes at 37 °C, followed by washing with normal saline solution. Then, the bead was removed into 500μl of 0.02M phosphate buffer containing peroxidase-labeled antibodies to mouse IgA and incubated for 15 minutes at 37 °C, followed by washing again with normal saline solution. Then, the bead was removed into 500μl of substrate solution (o-phenylenediamine solution containing hydrogen peroxide) and incubated for 15 minutes at 37 °C. Thereafter, the reaction was stopped by adding 3ml of 1.5N sulfuric acid solution. Absorbance at 492 nm of resulting solution was measured to quantitate the enzyme-activity of the enzyme bound to the bead.

30, 60 and 120 unit/ml standard solutions and 0 blank point were measured respectively in the same manner.

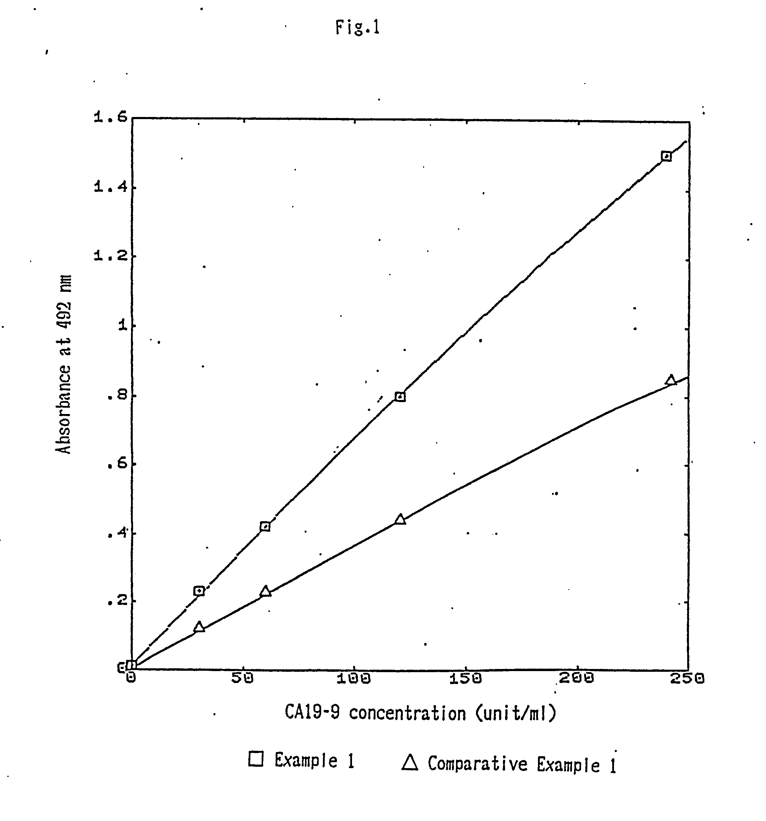

The result of measuring CA19-9 standard solution was shown in Fig.1.

Comparative Example 1 [Measurement of CA19-9 by EIA based on sandwich method]

Measurement of CA19-9 by enzyme immunoassay (EIA) based on sandwich method was performed to compare with example 1.

a) Producing monoclonal antibodies to CA19-9

Example 1, a) was repeated.

b) Producing glass beads conjugated monoclonal antibodies to CA19-9 (IgG1 subclass)

Example 1, b) was repeated.

c) Producing peroxidase-labeled monoclonal antibodies to CA19-9 (IgA class)

Monoclonal antibodies to CA19-9 (IgA class) were conbined with peroxidase as described in S.Yoshitake,M.Imagawa,E.Ishikawa,et.al.; J.Biochem.,Vol.92,1982,pp.1413-1424. The peroxidase-labeled monoclonal antibodies to CA19-9 (IgA class) were diluted to 1/10 - 1/5000 concentration with buffer containing 1% bovine serum albumin, and then used.

d) Producing CA19-9 standard solution

Example 1, d) was repeated.

e) Measuring CA19-9 standard solution

A glass bead conjugated monoclonal antibodies to CA19-9 (IgG1 subclass) was incubated with 50μl of 240 unit/ml standard solution and 400μl of 0.02M phosphate buffer containing 1% bovine serum albumin in a test tube for 15 minutes at 37 °C, followed by washing with normal saline solution. Then, the bead was removed into 500μl of 0.02M phosphate buffer containing peroxidase-labeled monoclonal antibodies to CA19-9 (IgA) and incubated for 15 minutes at 37 °C, followed by washing again with normal saline solution. Then, the bead was removed into 500μl of substrate solution (o-phenylenediamine solution containing hydrogen peroxide) and incubated for 15 minutes at 37 °C. Thereafter, the reaction was stopped by adding 3ml of 1.5N sulfuric acid solution. Absorbance at 492 nm of resulting solution was measured to quantitate the enzyme-activity of the enzyme bound to the bead.

30, 60 and 120 unit/ml standard solutions and 0 blank point were measured respectively in the same manner.

The result of measuring CA19-9 standard solution was shown in Fig.1.

Example 2 [Measurement of CEA ]

a) Producing monoclonal antibodies to CEA

Monoclonal antibodies to CEA were produced as described in Roberto S.Accolla, Stefan Carrel and Jean-Pierre Mach ; Proc.Natl.Acad.Sci.USA Vol.77,No.1,1980, pp.563-566. A BALB/c mouse was immunized by intraperitoneal injection of 15 μg CEA purified from liver metastases of human colon carcinoma with complete Freund's adjuvant. Two months later, the mouse was injected intravenously with 15 μg CEA and sacrificed after three days. After sacrifice, all spleen cells washed in RPMI1640 medium were mixed with $2 \times 10^7$ mouse myeloma cells (P3-NS1/1-Ag 4.1) and fused in 1ml of RPMI1640 medium included 42.5% polyethylene glycol 1540 and 7.5% dimethyl sulfoxide for 1 minute at 37 °C. After 1 minute, the cells suspension was diluted gradually with 5ml of RPMI1640 medium. After the cells were separated centrifugally and washed, HAT medium (RPMI1640 medium containing hypoxanthine, aminopterin, thymidine and 10% fetal bovine serum) was added to amount to 20 ml, and then the cells in HAT medium were seeded in 96 wells microplate. Two weeks later, culture media from wells containing growing hybrid cells were tested for the presence of antibody activity. The hybrid cells that had antibody activity were cloned repeatly at limiting dilution, then two clones of hybrid cells that produced IgG1 subclass monoclonal antibodies and IgG2a subclass monoclonal antibodies were obtained. These two clones of hybrid cells were injected intraperitoneally into mice and ascites fluid was obtained after hybrid cells grew as ascites tumors. The monoclonal antibodies in ascites fluid were purified with affi-gel-protein A MAPS kit (BIO-RAD).

b) Producing glass beads conjugated monoclonal antibodies to CEA (IgG2a subclass)

Monoclonal antibodies to CEA (IgG2a subclass) were coated onto the surface of glass beads as described in U.S. Pat.No.3652761.

c) Producing peroxidase-labeled antibodies to mouse IgG1

Antibodies to mouse IgG1 (Binding Site Ltd) were conbined with peroxidase as described in S.Yoshitake, M.Imagawa, E.Ishikawa,et.al.; J.Biochem.,Vol.92,1982,pp.1413-1424 The peroxidase-labeled antibodies to mouse IgG1 were diluted to 1/10 - 1/5000 concentration with buffer containing 1% bovine serum albumin, and then used.

d) Producing CEA standard solution

Concentration of CEA purified from liver metastases of human colon carcinoma by perchloric acid extraction was measured to compare with CEA International Reference Standard (63/701) with Glaozyme CEA kit (Sanyo Chemical Industriesl,Ltd.). 5, 10, 30 and 60 ng/ml standard solutions were produced by diluting the purified CEA with buffer containing 1% bovine serum albumin.

e) Producing CEA kits

One hundred glass beads conjugated monoclonal antibodies to CEA (IgG2a subclass), 12 ml of 0.02M phosphate buffer containing monoclonal antibodies to CEA (IgG1 subclass) at concentration of 10μg/ml, 60 ml of 0.02M phosphate buffer containing peroxidase-labeled antibodies to mouse IgG1, 40 ml of 0.02M phosphate buffer containing 1% bovine serum albumin, 1 ml of each standard solution (5, 10, 30 and 60 ng /ml), 1000 ml of normal saline solution, 60 ml of substrate solution (o-phenylenediamine solution containing hydrogen peroxide) and 350 ml of 1.5N sulfuric acid solution were bottled and stoppered respectively. A CEA kit consisted of these reagents.

f) Measuring CEA standard solution

A glass bead conjugated monoclonal antibodies to CEA (IgG2a subclass) was incubated with 50μl of 60 ng/ml standard solution, 100μl of 0.02M phosphate buffer containing monoclonal antibodies to CEA (IgG1 subclass) at concentration of 10μg/ml and 300 μl of 0.02M phosphate buffer containing 1% bovine serum albumin in a test tube for 15 minutes at 37 °C, followed by washing with normal saline solution. Then, the bead was removed into 500μl of 0.02M phosphate buffer containing peroxidase-labeled antibodies to mouse IgG1 and incubated for 15 minutes at 37 °C, followed by washing again with normal saline solution. Then, the bead was rem oved into 500μl of substrate solution (o-phenylenediamine solution containing hydrogen peroxide) and incubated for 15 minutes at 37 °C. Thereafter the reaction was stopped by adding 3ml of 1.5N sulfuric acid solution. Absorbance at 492 nm of resulting solution was measured to quantitate the enzyme-activity of the enzyme bound to the the bead.

5, 10 and 30 ng/ml standard solutions and 0 blank point were measured respectively in the same manner.

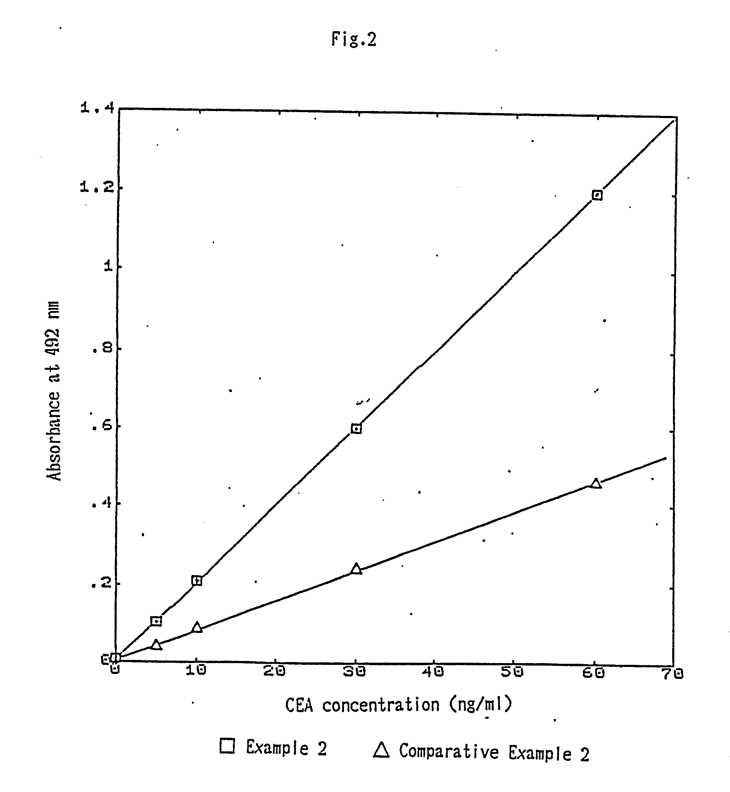

The result of measuring CEA standard solution was shown in Fig. 2.

Comparative Example 2 [Measurement of CEA by EIA based on sandwich method]

Measurement of CEA by enzyme immunoassay (EIA) based on sandwich method was performed to compare with example 2.

a) Producing monoclonal antibodies to CEA

Example 2, a) was repeated.

b) Producing glass beads conjugated monoclonal antibodies to CEA (IgG2a subclass)

Example 2, b) was repeated.

c) Producing peroxidase-labeled monoclonal antibodies to CEA (IgG1 subclass)

Monoclonal antibodies to CEA (IgG1 subclass) were conbined with peroxidase as described in S.Yoshitake,M.Imagawa,E.Ishikawa,et.al.; J.Biochem.,Vol.92,1982,pp.1413-1424. The peroxidase-labeled monoclonal antibodies to CEA (IgG1 subclass) were diluted to 1/10 - 1/5000 concentration with buffer containing 1% bovine serum albumin, and then used.

d) Producing CEA standard solution

Example 2, d) was repeated.

e) Measuring CEA standard solution

A glass bead conjugated monoclonal antibodies to CEA (IgG2a subclass) was incubated with 50μl of 60 ng/ml standard solution and 400μl of 0.02M phosphate buffer containing 1% bovine serum albumin in a test tube for 15 minutes at 37 °C, followed by washing with normal saline solution. Then, the bead was removed into 500μl of 0.02M phosphate buffer containing peroxidase-labeled monoclonal antibodies to CEA (IgG1) and incubated for 15 minutes at 37 °C, followed by washing again with normal saline solution. Then, the bead was removed into 500μl of substrate solution (o-phenylenediamine solution containing hydrogen peroxide) and incubated for 15 minutes at 37 °C. Thereafter, the reaction was stopped by adding 3ml of 1.5N sulfuric acid solution. Absorbance at 492 nm of resulting solution was measured to quantitate the enzyme-activity of the enzyme bound to the bead.

5, 10 and 30 ng/ml standard solutions and 0 blank point were measured respectively in the same manner.

The result of measuring CEA standard solution was shown in Fig. 2.

Example 3 [Measurement of HBs ]

a) Producing monoclonal antibodies to HBs

Monoclonal antibodies to HBs were produced as described in K.Kunitomo, S.Yahara, H.Saji and T.Hori; Vox Sanguinis, Vol.45, No.2, 1983, pp.104-111. A BALB/c mouse was immunized by intraperitoneal injection of 500 μg HBs antigen (The Green Cross Corp.) with complete Freund's adjuvant. Ten days later, the mouse was injected intravenously with 100 μg HBs antigen and sacri-

ficed after three days. After sacrifice, all spleen cells washed in RPMI1640 medium were mixed with $2 \times 10^7$ mouse myeloma cells (P3-NS1/1-Ag 4.1) and fused in 1ml of RPMI1640 medium included 42.5% polyethylene glycol 1540 and 7.5% dimethyl sulfoxide for 1 minute at 37°C. After 1 minute, the cells suspension was diluted gradually with 5ml of RPMI1640 medium. After the cells were separated centrifugally and washed, HAT medium (RPMI1640 medium containing hypoxanthine, aminopterin, thymidine and 10% fetal bovine serum) was added to amount to 20 ml, and then the cells in HAT medium were seeded in 96 wells microplate. Two weeks later, culture media from wells containing growing hybrid cells were tested for the presence of antibody activity. The hybrid cells that had antibody activity were cloned repeatly at limiting dilution, then two clones of hybrid cells that produced IgM class monoclonal antibodies and IgG2a subclass monoclonal antibodies were obtained. These two clones of hybrid cells were injected intraperitoneally into mice and ascites fluid was obtained after hybrid cells grew as ascites tumors. The monoclonal antibodies in ascites fluid were purified with affi-gel-protein A MAPS kit (BIO-RAD).

b) Producing glass beads conjugated monoclonal antibodies to HBs (Igm class)

Monoclonal antibodies to HBs (IgM class) were coated onto the surface of glass beads as described in U.S.Pat.No. 3652761.

c) Producing $^{125}$I-labeled antibodies to mouse IgG2a

Antibodies to mouse IgG2a (Binding Site Ltd) were conbined with $^{125}$I as described in Barbara B.Mishell, Stanley M.Shigi; SELECTED METHODS IN CELLULAR IMMUNOLOGY, 1980, pp.315-316, The $^{125}$I-labeled antibodies to mouse IgG2a were diluted to 1/10 - 1/5000 concentration with buffer containing 1% bovine serum albumin, and then used.

d) Producing HBs standard solution

0.5, 1, 2.5, 5 and 10 ng/ml standard solutions were produced by diluting HBs antigen (40μg/vial) obtained from Green Cross Corp. with 0.02M phosphate buffer.

e) Producing HBs kits

One hundred glass beads conjugated monoclonal antibodies to HBs (IgM class), 12 ml of 0.02M phosphate buffer containing monoclonal antibodies to HBs (IgG2a subclass) at concentration of 10μg/ml, 60 ml of 0.02M phosphate buffer containing $^{125}$I-labeled antibodies to mouse IgG2a, 40 ml of 0.02M phosphate buffer containing 1% bovine serum albumin, 1 ml of each standard solution (0.5, 1, 2.5, 5 and 10 ng/ml) and 1000 ml of normal saline solution were bottled and stoppered respectively. A HBs kit consisted of these reagents.

f) Measuring HBs standard solution

A glass bead conjugated monoclonal antibodies to HBs (Igm class) was incubated with 50μl of 10 ng/ml standard solution, 100μl of 0.02M phosphate buffer containing monoclonal antibodies to HBs (IgG2a subclass) at concentration of 10μg/ml and 300μl of 0.02M phosphate buffer containing 1% bovine serum albumin in a test tube for 15 minutes at 37°C, followed by washing with normal saline solution. Then, the bead was removed into 500μl of 0.02M phosphate buffer containing $^{125}$I-labeled antibodies to mouse IgG2a and incubated for 15 minutes at 37°C, followed by washing again with normal saline solution. Thereafter, radioactivity was determined in gamma counter to quantitate the $^{125}$I bound to the bead.

0.5, 1, 2.5 and 5 ng/ml standard solutions and 0 blank point were measured respectively in the same manner.

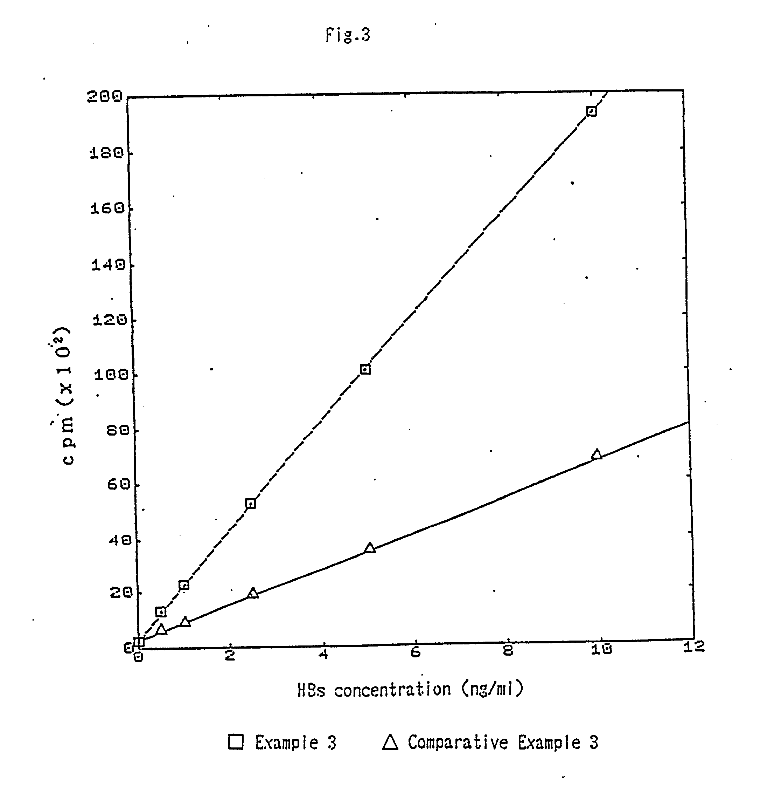

The result of measuring HBs standard solution was shown in Fig. 3.

Comparative Example 3 [Measurement of HBs by RIA based on sandwich method]

Measurement of HBs by radio immunoassay (RIA) based on sandwich method was performed to compare with example 3.

a) Producing monoclonal antibodies to HBs

Example 3, a) was repeated.

b) Producing glass beads conjugated monoclonal antibodies to HBs (IgM class)

Example 3, b) was repeated.

c) Producing $^{125}$I-labeled monoclonal antibodies to HBs (IgG2a subclass)

Monoclonal antibodies to HBs (IgG2a subclass) were conbined with $^{125}$I as described in Barbara B.Mishell, Stanley M.Shigi; SELECTED METHODS IN CELLULAR IMMUNOLOGY, 1980, pp.315-316. The $^{125}$I-labeld monoclonal antibodies to HBs (IgG2a subclass) were diluted to 1/10 - 1/5000 concentration with buffer containing 1% bovine serum albumin, and then used.

d) Producing HBs standard solution

Example 3, d) was repeated.

e) Measuring HBs standard solution

A glass bead conjugated monoclonal antibodies to HBs (IgM class) was incubated with 50μl of 10 ng/ml standard solution and 400μl of 0.02M phosphate buffer containing 1% bovine serum albumin in a test tube for 15 minutes at 37 °C, followed by washing with normal saline solution. Then, the bead was removed into 500μl of 0.02M phosphate buffer containing $^{125}$I-labeled monoclonal antibodies toHBs (IgG2a) and incubated for 15 minutes at 37 °C, followed by washing again with normal saline solution. Thereafter, radioactivity was determined in gamma counter to quantitate the $^{125}$I bound to the bead.

0.5, 1, 2.5 and 5 ng/ml standard solutions and 0 blank point were measured respectively in the same manner.

The result of measuring HBs standard solution was shown in Fig. 3.

Example 4 [Measurement of HCG ]

a) Producing monoclonal antibodies to HCG

Monoclonal antibodies to human chorionic gonadotropin(HCG) were produced as described in Gupta,S.K., Talwar,G.P.; Ind.J.Exp.Biol., Vol.13, 1980, pp.1361-1365. A BALB/c mouse was immunized by subcutaneous injection of 5 μg HCG antigen (Boehriger Mannheim) with incomplete Freund's adjuvant. Six weeks later, the mouse was injected intravenously with 200 μg HCG antigen in normal slaine solution and next every three days ,the mouse was injected intravenously and intraperitoneally with 400 μg HCG antigen in normal slaine solution and sacrificed at next day. After sacrifice, all spleen cells washed in RPMI1640 medium were mixed with 2x10⁷ mouse myeloma cells (P3-NS1/1-Ag 4.1) and fused in 1ml of RPMI 1640 medium included 42.5% polyethylene glycol 1540 and 7.5% dimethyl sulfoxide for 1 minute at 37 °C. After 1 minute, the cells suspension was diluted gradually with 5ml of RPMI1640 medium. After the cells were separated centrifugally and washed, HAT medium (RPMI1640 medium containing hypoxanthine, aminopterin, thymidine and 10% fetal bovine serum) was added to amount to 20ml, and then the cells in HAT medium were seeded in 96 wells microplate. Two weeks later, culture media from wells containing growing hybrid cells were tested for the presence of antibody activity. The hybrid cells that had antibody activity were cloned repeatly at limiting dilution, then two clones of hybrid cells that produced IgG2b subclass monoclonal antibodies and IgG1 subclass monoclonal antibodies were obtained. These two clones of hybrid cells were injected intraperitoneally into mice and ascites fluid was obtained after hybrid cells grew as ascites tumors. The monoclonal antibodies in ascites fluid were purified with affi-gel-protein A MAPS kit (BIO-RAD).

b) Producing glass beads conjugated monoclonal antibodies to HCG (IgG2b subclass)

Monoclonal antibodies to HCG (IgG2b subclass) were coated onto the surface of glass beads as described in U.S.Pat.No. 3652761.

c) Producing $^{125}$I-labeled antibodies to mouse IgG1

Antibodies to mouse IgG1 (Binding Site Ltd) were conbined with $^{125}$I as described in Barbara B.Mishell, Stanley M.Shigi; SELECTED METHODS IN CELLULAR IMMUNOLOGY, 1980, pp.315-316. The $^{125}$I-labeled antibodies to mouse IgG2a were diluted to 1/10 - 1/5000 concentration with buffer containing 1% bovine serum albumin, and then used.

d) Producing HCG standard solution

5, 10, 25, 50, 100 and 200 IU/l standard solutions were produced by diluting HCG antigen (10000IU/mg) obtained from Boehringer Mannheim with 0.02M phosphate buffer.

e) Producing HCG kits

One hundred glass beads conjugated monoclonal antibodies to HCG (IgG2b subclass), 12 ml of 0.02M phosphate buffer containing monoclonalantibodies to HCG (IgG1 subclass) at concentration of 10μg/ml, 60 ml of 0.02M phosphate

buffer containing $^{125}$I-labeled antibodies to mouse IgG1, 40 ml of 0.02M phosphate buffer containing 1% bovine serum albumin, 1 ml of each standard solution (5, 10, 25, 50, 100 and 200 IU/l) and 1000 ml of normal saline solution were bottled and stoppered respectively. A HCG kit consisted of these reagents.

f) Measuring HCG standard solution

A glass bead conjugated monoclonal antibodies to HCG (IgG2b subclass) was incubated with 50μl of 200 IU/l standard solution, 100μl of 0.02M phosphate buffer containing monoclonal antibodies to HCG (IgG1 subclass) at concentration of 10μg/ml and 300μl of 0.02M phosphate buffer containing 1% bovine serum albumin in a test tube for 15 minutes at 37 °C followed by washing with normal saline solution. Then, the bead was removed into 500μl of 0.02M phosphate buffer containing $^{125}$I-labeled antibodies to mouse IgG1 and incubated for 15 minutes at 37 °C, followed by washing again with normal saline solution. Thereafter, radioactivity was determined in gamma counter to quantitate the $^{125}$I bound to the the bead.

5, 10, 25, 50 and 100 IU/l standard solutions and 0 blank point were measured respectively in the same manner.

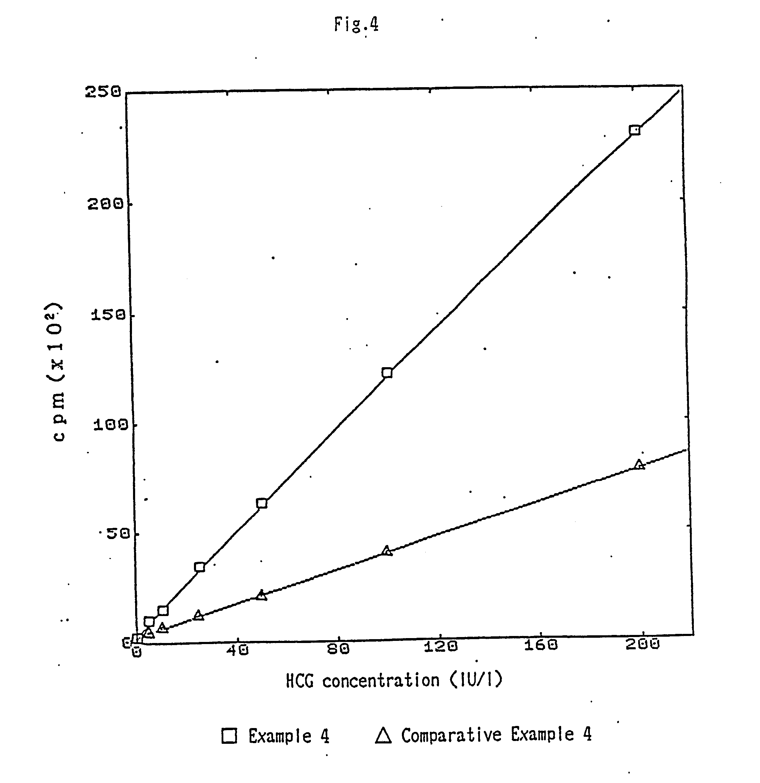

The result of measuring HCG standard solution was shown in Fig. 4.

Comparative Example 4 [Measurement of HCG by RIA based on sandwich method]

Measurement of HCG by radio immunoassay-(RIA) based on sandwich method was performed to compare with example 4.

a) Producing monoclonal antibodies to HCG

Example 4, a) was repeated.

b) Producing glass beads conjugated monoclonal antibodies to HCG (IgG2b subclass)

Example 4, b) was repeated.

c) Producing $^{125}$I-labeled monoclonal antibodies to HCG (IgG1 subclass)

Monoclonal antibodies to HCG (IgG1 subclass) were conbined with $^{125}$I as described in Barbara B.Mishell, Stanley M.Shigi; SELECTED METHODS IN CELLULAR IMMUNOLOGY, 1980, pp.315-316. The $^{125}$I-labeled monoclonal antibodies to HCG (IgG1 subclass) were diluted to 1/10 - 1/5000 concentration with buffer containing 1% bovine serum albumin, and then used.

d) Producing HCG standard solution

Example 4, d) was repeated.

e) Measuring HCG standard solution

A glass bead conjugated monoclonal antibodies to HCG (IgG2b subclass) was incubated with 50μl of 200 IU/l standard solution and 400 μl of 0.02M phosphate buffer containing 1% bovine serum albumin in a test tube for 15 minutes at 37 °C, followed by washing with normal saline solution. Then, the bead was removed into 500μl of 0.02M phosphate buffer containing $^{125}$I-labeled monoclonal antibodies to HCG (IgG1) and incubated for 15 minutes at 37 °C followed by washing again with normal saline solution. Thereafter, radioactivity was determined in gamma counter to quantitate the $^{125}$I bound to the bead.

5, 10, 25, 50 and 100 IU/l standard solutions and 0 blank point were measured respectively in the same manner.

The result of measuring HCG standard solution was shown in Fig. 4.

Example 5 [Measurement of CA19-9]

a) Producing monoclonal antibodies to CA19-9

Example 1, a) was repeated.

b) Exercising Fc fragments of monoclonal antibodies to CA19-9

20 mg of monoclonal antibodies to CA19-9 were added 0.4 mg of pepsin (Boehringer Mannheim) and incubated for 6 hours at 37 °C, after dialyzed with 0.1M NaCl-0.1M sodium acetate buffer (pH4.5). Then, Fc fragments were removed by gel filtration with ultrogel AcA44 (IBF Bio Technics) column (1.5cmx90cm) after the solution was adjusted to pH of 7.0 with 0.1 M sodium hydroxide. Furthermore, F(ab')2 fragments were purified by removing monoclonal antibodies that weren't excised with affi-gel-protein A MAPS kit.

c) Producing glass beads conjugated monoclonal antibodies to CA19-9 excised Fc fragments

Monoclonal antibodies to CA19-9 excised Fc fragments were coated onto the surface of glass beads as described in U.S. Pat.No. 3652761.

d) Producing peroxidase-labeled antibodies to mouse IgG Fc fragments

Antibodies to mouse IgG Fc fragments (Binding Site Ltd) were conbined with peroxidase as described in S.Yoshitake,M.Imagawa,E.Ishikawa,et.al.; J.Biochem.,Vol.92,982,pp.1413-1424. The peroxidase-labeled antibodies to mouse IgG Fc fragments were diluted to 1/10 - 1/5000 concentration with buffer containing 1% bovine serum albumin, and then used.

e) Producing CA19-9 standard solution

Example 1, d) was repeated.

f) Producing CA19-9 kits

One hundred glass beads conjugated monoclonal antibodies to CA19-9 excised Fc fragments, 12 ml of 0.02M phosphate buffer containing monoclonal antibodies to CA19-9 (IgG class) at concentration of 10μg/ml, 60 ml of 0.02M phosphate buffer containing peroxidase-labeled antibodies to mouse IgG Fc fragments, 40 ml of 0.02M phosphate buffer containing 1% bovine serum albumin, 1 ml of each standard solution (30, 60, 120 and 240 unit/ml), 1000 ml of normal saline solution, 60 ml of substrate solution (o-phenylenediamine solution containing hydrogen peroxide) and 350 ml of 1.5N sulfuric acid solution were bottled and stoppered respectively. A CA19-9 kit consisted of these reagents.

g) Measuring CA19-9standard solution

A glass bead conjugated monoclonal antibodies to CA19-9 excised Fc fragments was incubated with 50μl of 240 unit/ml standard solution, 100μl of 0.02M phosphate buffer containing monoclonal antibodies to CA19-9 (IgG class) at concentration of 10μg/ml and 300μl of 0.02M phosphate buffer containing 1% bovine serum albumin in a test tube for 15 minutes at 37 °C, followed by washing with normal saline solution. Then, the bead was removed into 500μl of 0.02M phosphate buffer containing peroxidase-labeled antibodies to mouse IgG Fc fragments and incubated for 15 minutes at 37 °C, followed by washing again with normal saline solution. Then, the bead was removed into 500μl of substrate solution (o-phenylenediamine solution containing hydrogen peroxide) and incubated for 15 minutes at 37 °C. Thereafter, the reaction was stopped by adding 3ml of 1.5N sulfuric acid solution. Absorbance at 492 nm of resulting solution was measured to quantitate the enzyme-activity of the enzyme bound to the bead.

30, 60 and 120 unit/ml standard solutions and 0 blank point were measured respectively in the same manner.

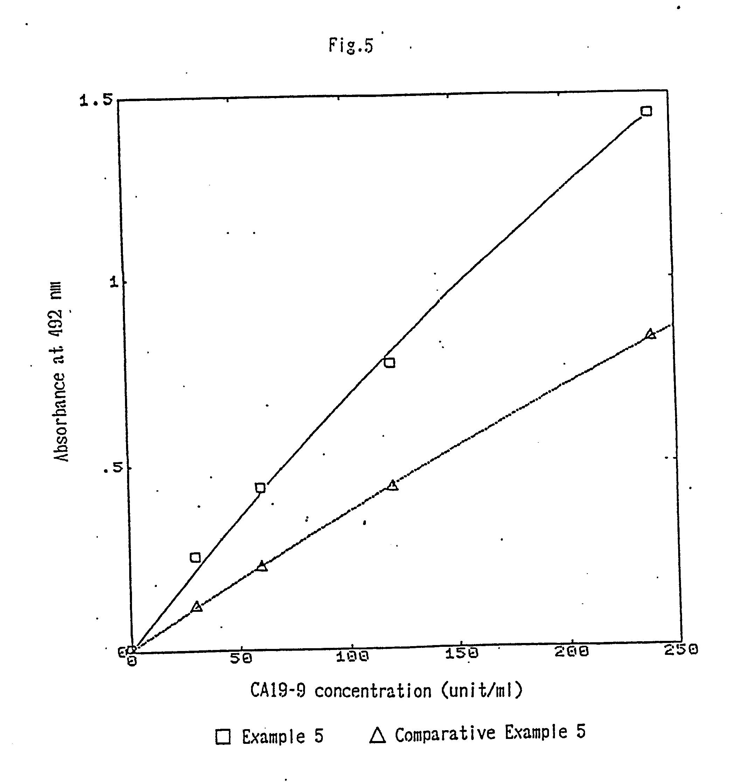

The result of measuring CA19-9 standard solution was shown in Fig.5.

Comparative Example 5 [Measurement of CA19-9 by EIA based on sandwich method]

Measurement of CA19-9 by enzyme immunoassay (EIA) based, on sandwich method was performed to compare with example 5.

a) Producing monoclonal antibodies to CA19-9

Example 1, a) was repeated.

b) Producing glass beads conjugated monoclonal antibodies to CA19-9

Example 1, b) was repeated.

c) Producing peroxidase-labeled monoclonal antibodies to CA19-9

Monoclonal antibodies to CA19-9 were conbined with peroxidase as described in S.Yoshitake, M.Imagawa, E.Ishikawa,et.al.; J.Biochem.,Vol.92,1982,pp.1413-1424 The peroxidase-labeled monoclonal antibodies to CA19-9 were diluted to 1/10 - 1/5000 concentration with buffer containing 1% bovine serum albumin, and then used.

d) Producing CA19-9 standard solution

Example 1, d) was repeated.

e) Measuring CA19-9 standard solution

A glass bead conjugated monoclonal antibodies to CA19-9 was incubated with 50μl of 240 unit/ml standard solution and 400μl of 0.02M phosphate buffer containing 1% bovine serum albumin in a test tube for 15 minutes at 37 °C, followed by washing with normal saline solution. Then, the bead was removed into 500μl of 0.02M phosphate buffer containing peroxidase-labeled monoclonal antibodies to CA19-9 and incubated for 15 minutes at 37 °C, followed by washing again with normal saline solution. Then, the bead was removed into 500μl of substrate solution (o-phenylenediamine solution containing hydrogen peroxide) and incubated for 15 minutes at 37 °C. Thereafter, the reaction was stopped by adding 3ml of 1.5N sulfuric acid solution. Absorbance at 492 nm of resulting solution was measured to quantitate the enzyme-activity of the enzyme bound to the bead.

30, 60 and 120 unit/ml standard solutions and 0 blank point were measured respectively in the same manner.

The result of measuring CA19-9 standard solution was shown in Fig.5.

Example 6 [Measurement of HBs ]

a)Producing monoclonal antibodies to HBs

Example 3, a) was repeated.

b) Excising Fc fragments of monoclonal antibodies to HBs

20 mg of monoclonal antibodies to HBs were added 0.4 mg of pepsin (Boehringer Mannheim) and incubated for 6 hours at 37 °C after dialyzed with 0.1M NaCl-0.1M sodium acetate buffer (pH4.5). Then, Fc fragments were removed by gel filtration with ultrogel AcA44 (IBF Bio Technics) column (1.5cmx90cm) after the solution was adjusted to pH of 7.0 with 0.1 M sodium hydroxide. Furthermore, F(ab′)₂ fragments were purified by removing monoclonal antibodies that weren't excised with affi-gel-protein A MAPS kit.

c) Producing glass beads conjugated monoclonal antibodies to HBs (IgG class) excised Fc fragments

Monoclonal antibodies to HBs (IgG class) excised Fc fragments were coated onto the surface of glass beads as described in U.S.Pat.No.3652761.

d) Producing ¹²⁵I-labeled antibodies to mouse IgG Fc fragments

Antibodies to mouse IgG Fc fragments (Binding Site Ltd) were conbined with ¹²⁵I as described in Barbara B.Mishell, Stanley M. Shigi; SELECTED METHODS IN CELLULAR IMMUNOLOGY, 1980, pp.315-316. The ¹²⁵I-labeled antibodies to mouse IgG Fc fragments were diluted to 1/10 - 1/5000 concentration with buffer containing 1% bovine serum albumin, and then used.

e) Producing HBs standard solution

Example 3, d) was repeated.

f) Producing HBs kits

One hundred glass beads conjugated monoclonal antibodies to HBs (IgG class) excised Fc fragments, 12 ml of 0.02M phosphate buffer containing monoclonal antibodies to HBs (IgG class) at concentration of 10μg/ml, 60 ml of 0.02M phosphate buffer containing ¹²⁵I-labeled antibodies to mouse IgG Fc fragments, 40 ml of 0.02M phosphate buffer containing 1% bovine serum albumin, 1 ml of each standard solution (0.5, 1, 2.5, 5 and 10 ng/ml) and 1000 ml of normal saline solution were bottled and stoppered respectively. A HBs kit consisted of these reagents.

g) Measuring HBs standard solution

A glass bead conjugated monoclonal antibodies to HBs (IgG class ) excised Fc fragments was incubated with 50μl of 10 ng/ml standard solution, 100μl of 0.02M phosphate buffer containing monoclonal antibodies to HBs (IgG class) at concentration of 10μg/ml and 300μl of 0.02M phosphate buffer containing 1% bovine serum albumin in a test tube for 15 minutes at 37 °C, followed by washing with normal saline solution. Then, the bead was removed into 500 μl of 0.02M phosphate buffer containing ¹²⁵I-labeled antibodies to mouse IgG Fc fragments and incubated for 15 minutes at 37 °C, followed by washing again with normal saline solution. Thereafter, radioactivity was determined in gamma counter to quantitate the ¹²⁵I bound to the the bead.

0.5, 1, 2.5 and 5 ng/ml standard solutions and 0 blank point were measured respectively in the same manner.

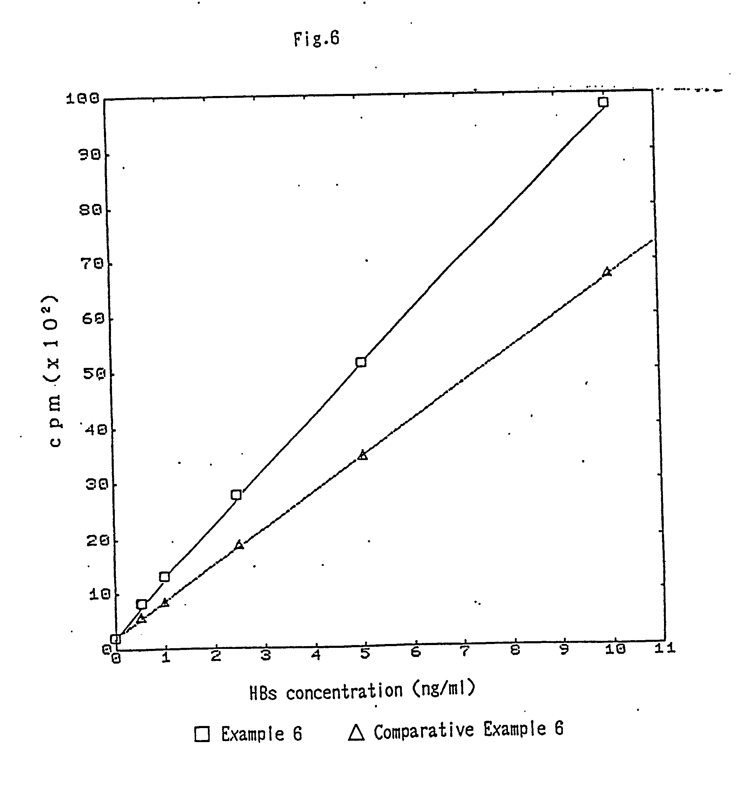

The result of measuring HBs standard solution was shown in Fig. 6.

Comparative Example 6 [Measurement of HBs by RIA based on sandwich method]

Measurement of HBs by radio immunoassay-(RIA) based on sandwich method was performed to compare with example 6.

a) Producing monoclonal antibodies to HBs

Example 3, a) was repeated.

b) Producing glass beads conjugated monoclonal antibodies to HBs (IgG class)

Example 3, b) was repeated.

c) Producing 125I-labeled monoclonal antibodies to HBs(IgG class)

Monoclonal antibodies to HBs (IgG class) were conbined with 125I as described in Barbara B.Mishell, Stanley M.Shigi; SELECTED METHODS IN CELLULAR IMMUNOLOGY, 1980, pp.315-316. The 125I-labeled monoclonal antibodies to HBs (IgG class) were diluted to 1/10 - 1/5000 concentration with buffer containing 1% bovine serum albumin, and then used.

d) Producing HBs standard solution

Example 3, d) was repeated.

e) Measuring HBs standard solution

A glass bead conjugated monoclonal antibodies to HBs (IgG class) was incubated with 50μl of 10 ng/ml standard solution and 400μl of 0.02M phosphate buffer containing 1% bovine serum albumin in a test tube for 15 minutes at 37 °C, followed by washing with normal saline solution. Then, the bead was removed into 500μl of 0.02M phosphate buffer containing 125I-labeled monoclonal antibodies to HBs (IgG class) and incubated for 15 minutes at 37 °C followed by washing again with normal saline solution. Thereafter, radioactivity was determined in gamma counter to quantitate the 125I bound to the bead.

0.5, 1, 2.5 and 5 ng/ml standard solutions and 0 blank point were measured respectively in the same manner.

The result of measuring HBs standard solution was shown in Fig. 6.

It will be seen that the present invention provides an immunoassay of proved sensitivity, a high sensitivity immunoassay, even when monoclonal antibodies of poor affinity is used, and an immunoassay which can attain improved sensitivity without lowering precision.

**Claims**

1. An immunoassay method comprising:
(1) reacting a material to be measured, first, immobilized, monoclonal antibody recognizing the material, a second monoclonal antibody recognizing the material and falling within a different class or subclass from the first antibody, and a third, labelled, antibody recognizing the class or subclass of the second antibody to obtain an immuno-complex; and (2) detecting or determining the labelling fragment.

2. An immonoassay method according to claim 1, in which the first antibody and the second antibody originate from the same enimal species.

3. An immunoassay method, comprising:
(1) reacting a material to be measured, a first binding protein to bind specifically to the material, a second, immobilized, binding protein comprising the rest of the first protein, part of which has been used away, and a labelled antibody recognizing all or part of the excised fragment of the first binding protein to obtain an immuno-complex; and (2) detecting or determining the labelling fragment.

4. An immonoassay method according to claim 3, in which the first binding protein is a monoclonal antibody.

5. An immunoassay method according to claim 3, in which the first binding protein is selected from polyclonal antibody, enzymes, enzyme inhibitors, hormones, cytokines, receptors and lectin.

6. An immunoassay method according to any of claims 3-5, in which the excised fragment is a Fc fragment.

7. An immunoassay method according to any preceding claim, in which the material is selected from hormones, enzymes, serum-proteins, tumor-associated antigens, DNA binding protein factors, cytokines, microorganisms, virus and protozoans.

8. An immunoassay method according to any preceding claim, in which the material is insulin, HCG-beta, growth hormone, TSH, thyroxine, triiodothyronine, gastrin, glucagon, somatostatin, elastase, amylase, proteinase, lipase, ribonuclease, IgG, IgA, IgM, IgE, IgD, glycoproteins, $beta_2$-micro-globulin, TBG, glycolipids, CEA, alphafetoprotein, ferritin, POA, CA19-9, CA125, DNA binding protein factors, cytokine, interferon, interleukin 1, interleukin 2, eumycetes, streptococcus, hepatitis virus, herpes virus, AIDS virus, Toxoplasma Nicoll-Manceau, plasmodium or Entamoeba histolytica Schaudinn.

9. An immunoassay method according to any preceding claim, in which the material has at least two equivalent binding sites.

10. An immunoassay method according to any preceding claim, in which the first antibody or second binding protein is immobilized on an insoluble material selected from siliceus carriers, magnetic carriers and organic carriers.

11. An immunoassay method according to any preceding claim, in which the third antibody or labelled antibody which recognises all or part of the excised fragment has been labelled with a marker selected from isotopes, enzymes, fluorescent chromophores and chemical luminescent chromophores.

12. A kit for the detection or determination of a material to be measured, comprising:

1) a first immobolized, monoclonal antibody recognizing the material,

2) a second monoclonal antibody recognizing the material and falling within a different class or subclass from the first antibody, and

3) a labelled, third, antibody recognizing the class or subclass of the second antibody.

13. A kit for the detection or determination of a material to be measured, comprising:

1) a first binding protein to bind specifically to the material,

2) a second, immobilized, binding protein comprising the rest of the 1st binding protein, a part of which has been excised away, and

3) a labelled antibody recognizing all or part of the excised fragment of the first binding protein.

Fig.1

CA19-9 concentration (unit/ml)

□ Example 1     △ Comparative Example 1

Fig.2

CEA concentration (ng/ml)

☐ Example 2     △ Comparative Example 2

Fig.3

□ Example 3     △ Comparative Example 3

Fig.4

□ Example 4    △ Comparative Example 4

Fig.5

CA19-9 concentration (unit/ml)

□ Example 5     △ Comparative Example 5

Fig.6

HBs concentration (ng/ml)

☐ Example 6    △ Comparative Example 6